# EUROPÄISCHE PATENTSCHRIFT

(11) **EP 1 732 953 B1**
(45) Veröffentlichungstag und Bekanntmachung des Hinweises auf die Patenterteilung: **07.11.2007**
(21) Anmeldenummer: 05708068.1
(22) Anmeldetag: 01.03.2005
(51) Int. Cl.: C08B 31/10, A61K 47/36, A61K 31/718, A61B 10/00

(54) **HYDROXYETHYLSTÄRKE**
HYDROXYETHYL STARCH
HYDROXYETHYLAMIDON

(30) Priorität: 01.03.2004 EP 04100813
(43) Veröffentlichungstag der Anmeldung: 20.12.2006
(73) Patentinhaber: B. BRAUN MELSUNGEN AG, 34212 Melsungen (DE)
(72) Erfinder: BOLL, Michael, 34212 Melsungen (DE); FISCH, Andreas, 1005 Lausanne (CH); SPAHN, Donat R., CH-8053 Zürich (CH)
(74) Vertreter: Weber, Thomas
(86) Internationale Anmeldenummer: PCT/EP2005/050877
(87) Internationale Veröffentlichungsnummer: WO 2005/082942

(56) Entgegenhaltungen:
- EP-A- 0 170 275
- EP-A- 0 402 724
- WO-A-00/48637
- GB-A- 1 395 777
- US-A- 5 502 043
- US-B1- 6 680 305
- A.N. DE BELDER AND B. NORRMAN: "The substitution patterns of O-(2-hydroxyethyl)starch and O-(2-hydroxyethyl)dextran" CARBOHYDRATE RESEARCH, Bd. 10, 1969, Seiten 391-394, XP002329873 AMSTERDAM

## Beschreibung

Die Erfindung betrifft eine Hydroxyethylstärke sowie ein Verfahren zur Herstellung einer solchen Hydroxyethylstärke. Weiterhin betrifft die Erfindung eine pharmazeutische Zubereitung enthaltend eine Hydroxyethylstärke sowie die Verwendung einer solchen pharmazeutischen Zubereitung zur Herstellung eines Volumenersatzmittels, eines Plasmaersatzmittels oder eines Plasmaexpanders als auch die Verwendung der pharmazeutischen Zubereitung zur Aufrechterhaltung der Normovolämie und/oder zur Verbesserung der Makro- und Mikrozirkulation und/oder zur Verbesserung der nutritiven Sauerstoffversorgung und/oder zur Stabilisierung der Hämodynamik und/oder zur Verbesserung der Volumenwirksamkeit und/oder zur Verringerung der Plasmaviskosität und/oder zur Erhöhung der Anämietoleranz und/oder zur Hämodilution, insbesondere zur therapeutischen Hämodilution bei Durchblutungsstörungen und arteriellen, speziell peripheren arteriellen, Verschlusskrankheiten.

Der Ersatz intravasaler Flüssigkeit gehört zu den wichtigsten Maßnahmen bei der Prophylaxe und Therapie der Hypovolämie und zwar unabhängig davon, ob die Hypovolämie aus dem unmittelbaren Verlust von Blut bzw. Körperflüssigkeiten resultiert (bei akuten Blutungen, Traumen, Operationen, Verbrennungen), aus Verteilungsstörungen zwischen Makro- und Mikrozirkulation (wie bei Sepsis) oder aus einer Vasodilation (z. B. bei der Anästhesieeinleitung). Für diese Indikationen geeignete Infusionslösungen sollen die Normovolämie wieder herstellen und die Perfusion lebenswichtiger Organe sowie den peripheren Blutfluss aufrechterhalten. Gleichzeitig dürfen die Lösungen den Kreislauf nicht übermäßig belasten und sie müssen von Nebenwirkungen möglichst frei sein. In dieser Hinsicht bieten sämtliche derzeit verfügbaren Volumenersatzlösungen Vor- und Nachteile. Sogenannte kristalloide Lösungen (Elektrolytlösungen) sind zwar weitgehend frei von unmittelbaren Nebenwirkungen, gewährleisten aber nur kurzfristige oder inadäquate Stabilisierung des intravasalen Volumens und der Hämodynamik. Bei ausgeprägter oder länger anhaltender Hypovolämie müssen sie in exzessiven Mengen infundiert werden, da sie nicht ausschließlich im intravasalen Kompartiment verbleiben, sondern sich rasch im extravasalen Raum verteilen. Ein schneller Abstrom in den extravasalen Raum limitiert aber nicht nur die kreislauffüllende Wirkung kristalloider Lösungen, sondern birgt auch das Risiko peripherer und pulmonaler Ödeme. Abgesehen von der vitalen Bedrohung, die ein Lungenödem darstellen kann, führt es außerdem zur Verschlechterung der nutritiven Sauerstoffversorgung, die durch periphere Ödeme ebenfalls beeinträchtigt wird.

Demgegenüber sind kolloidale Volumenersatzlösungen, seien die in ihnen enthaltenen Kolloide natürlichen oder synthetischen Ursprungs, in ihrer Wirkung weitaus zuverlässiger. Dies ist darauf zurückzuführen, dass sie aufgrund ihrer kolloidosmotischen Wirkung die zugeführte Flüssigkeit länger im Kreislauf zurückhalten als Kristalloide und sie so vor dem Abstrom in das Interstitium schützen. Andererseits geben kolloidale Volumenersatzlösungen in höherem Maß als kristalloide Lösungen zu unerwünschten Reaktionen Anlass. So birgt das natürliche Kolloid Albumin, wie alle Blut- bzw. Plasmaderivate, das Risiko der Ansteckung mit viralen Erkrankungen; zusätzlich kann es zu Wechselwirkungen mit anderen Arzneimitteln, z. B. ACE-Inhibitoren, kommen; schließlich ist die Verfügbarkeit von Albumin limitiert und seine Verwendung als Volumenersatzmittel unverhältnismäßig teuer. Weitere Bedenken gegen den Einsatz von Albumin als Volumenersatzmittel haben ihren Grund in der Hemmung der endogenen Synthese von Albumin bei dessen exogener Zufuhr und in seiner leichten Extravasation. Hierunter wird der Übertritt aus dem Kreislauf in den extravasalen Raum verstanden, wo es wegen des kolloidosmotischen Effekts von Albumin zu unerwünschten und langanhaltenden Flüssigkeitsansammlungen kommen kann.
Bei den synthetischen Kolloiden haben schwere anaphylaktoide Reaktionen und eine massive Beeinträchtigung der Blutgerinnung dazu geführt, dass Dextran-Präparate aus der Therapie nahezu völlig verschwunden sind. Hydroxyethylstärke (HES)-Lösungen besitzen zwar ebenfalls das Potential zur Auslösung anaphylaktoider Reaktionen und zur Beeinflussung der Blutgerinnung, dies jedoch in geringerem Maß als Dextran. Schwerwiegende anaphylaktoide Reaktionen (Reaktionen der Schweregrade III und IV) werden mit HES-Lösungen - anders als mit Dextran - äußerst selten beobachtet; und der Einfluss auf die Blutgerinnung, der den hochmolekularen HES-Lösungen eigen ist, konnte durch die Weiterentwicklung von HES-Lösungen in den letzten Jahren erheblich vermindert werden. Im Vergleich mit den Gelatinelösungen, die ebenfalls als Plasmaersatzmittel Verwendung finden und die Blutgerinnung weitgehend unbeeinflusst lassen, besitzen HES-Lösungen, zumindest deren hoch- und mittelmolekulare Ausführungsformen, den Vorzug längerer Plasmaverweildauer und Wirksamkeit.

EP-A-0402724 offenbart die Herstellung und Verwendung einer Hydroxyethylstärke mit einem mittleren Molekulargewicht Mw von 60000 bis 600000, einem molaren Substitutionsgrad MS von 0,15 bis 0,5 und einem Substitutionsgrad DS von 0,15 bis 0,5. Die Offenbarung beschäftigt sich mit der schnellen (6 bis 12 Stunden) und vollständigen Abbaubarkeit der als Plasmaexpander einzusetzenden Hydroxyethylstärken. Explizit untersucht wurde dabei innerhalb des bevorzugten mittleren Molekulargewichtsbereichs von 100000 bis 300000 eine Hydroxyethylstärke mit einem mittleren Molekulargewicht von 234000.

US-A-5,502,043 offenbart die Verwendung von Hydroxyethylstärken mit einem mittleren Molekulargewicht Mw von 110000 bis 150000, einer molaren Substitution MS von 0,38 bis 0,5 und einem Substitutionsgrad DS von 0,32 bis 0,45 zur Verbesserung der Mikrozirkulation bei peripherer arterieller Durchblutungsstörung. Darüber hinaus lehrt die Druckschrift den Einsatz von niedermolekularen (Mw 110000 bis 150000) Hydroxyethylstärken, die bedingt durch ihr niedriges Molekulargewicht die Plasmaviskosität niedrig halten und somit eine Verbesserung der Mikrozirkulation im Blutkreislauf gewährleisten. Abgeraten wird in dieser Schrift jedoch von dem Einsatz höhermolekularer Hydroxyethylstärken, wie etwa einer Hydroxyethylstärke mit einem Mw: 500000, da diese trotz niedriger molarer Substitution (MS=0,28) die Plasmaviskosität erhöhen und somit die Mikrozirkulation verschlechtern.

Weltweit werden zur Zeit unterschiedliche HES-Präparate als kolloidale Volumenersatzmittel verwendet, die sich hauptsächlich in ihrem Molekulargewicht und daneben in dem Ausmaß der Veretherung mit Hydroxyethylgruppen und in anderen Parametem unterscheiden. Die bekanntesten Vertreter dieser Substanzklasse sind die sog. Hetastarch (HES 450/0,7) und Pentastarch (HES 200/0,5). Bei letztgenannter handelt es sich um die derzeit am weitesten verbreitete "Standard-HES". Neben dieser spielen HES 200/0,62 und HES 70/0,5 eine geringere Rolle. Bei den deklarierten Angaben zum Molekulargewicht handelt es sich ebenso wie bei denjenigen zu den anderen Parametern um gemittelte Größen, wobei der Molekulargewichtsdeklaration das Massenmittel (Mw) zugrunde liegt, das in Dalton (z. B. bei HES 200000) oder meist verkürzt in Kilodalton (z. B. wie bei HES 200) angegeben wird. Das Ausmaß der Veretherung mit Hydroxyethylgruppen wird durch die molare Substition MS (z.B. als 0,5 wie in HES 200/0,5; MS = durchschnittliches molares Verhältnis von Hydroxyethylgruppen zu Anhydroglucoseeinheiten) oder durch den Substitutionsgrad (DS = Verhältnis von ein- oder mehrfach hydroxyethylierten Glucosen zu den Gesamt-Anhydroglucoseeinheiten) gekennzeichnet. Entsprechend ihrem Molekulargewicht werden die in klinischem Gebrauch befindlichen HES-Lösungen in hochmolekulare (450 kD), mittelmolekulare (200 - 250 kD) und niedermolekulare Präparate (70 - 130 kD) eingeteilt.

Was die Gerinnungseffekte von HES-Lösungen angeht, so ist zwischen unspezifischen und spezifischen Einflüssen zu unterscheiden. Zu einer unspezifischen Beeinflussung der Blutgerinnung kommt es aufgrund der Verdünnung des Bluts (Hämodilution), die bei der Infusion von HES- und anderen Volumenersatzlösungen in den Blutkreislauf stattfindet. Von dieser Hämodilution sind auch Gerinnungsfaktoren betroffen, deren Konzentrationen je nach Ausmaß und Dauer der infusionsbedingten Verdünnung des Bluts und der Plasmaproteine absinken. Entsprechend große oder anhaltende Effekte können zu einer labordiagnostisch nachweisbaren, in extremen Fällen zu einer klinisch relevanten Hypokoagulabilität führen.

Daneben kann von der Hydroxyethylstärke eine spezifische Beeinflussung der Blutgerinnung ausgehen, für die mehrere Faktoren verantwortlich gemacht werden. So findet man - unter bestimmten Bedingungen bzw. mit bestimmten HES-Präparaten - einen Abfall der Gerinnungsproteine Faktor VIII (F VIII) und von Willebrand-Faktor (vWF), der größer ist als die hämodilufionsbedingte Abnahme der Plasmaproteine im allgemeinen. Ob dieser stärkere als zu erwartende Abfall durch eine verminderte Bildung bzw. Freisetzung von F VIII/vWF bedingt ist, etwa durch HES-bedingte Coating-Effekte am Gefäßendothel oder durch andere Mechanismen, ist nicht völlig geklärt.

HES beeinflusst aber nicht nur die Konzentration der genannten Gerinnungsfaktoren, sondern offenbar auch die Funktion der Blutplättchen. Hierfür ist ganz oder teilweise die Bindung von HES an die Oberfläche der Blutplättchen verantwortlich, die den Zutritt von Liganden an den Fibrinogenrezeptor der Plättchen hemmt.

Diese spezifischen Wirkungen von HES auf die Blutgerinnung sind bei der Anwendung hochmolekularer HES (z. B. von HES 450/0,7) besonders ausgeprägt, während sie bei mittelmolekularer (z. B. HES 250/0,5) oder niedermolekularer HES (z. B. HES 130/0,4 oder HES 70/0,5) keine so große Rolle mehr spielen (J. Treib et al., Intensive Care Med. (1999), S. 258 bis 268; O. Langeron et al., Anesth. Analg. (2001), S. 855 bis 862; R.G. Strauss et al., Transfusion (1988), S. 257 - 260; M. Jamnicki et al., A-nesthesiology (2000), S. 1231 bis 1237).

Vergleicht man das Risikoprofil hochmolekularer HES mit dem der mittel- und niedermolekularen Präparate, so kann man bei den letztgenannten eine deutliche Reduzierung der Risiken feststellen, und zwar nicht nur im Hinblick auf die Wechselwirkung mit der Blutgerinnung, sondern auch im Hinblick auf bestimmte pharmakokinetische Eigenschaften. So zeigen die hochmolekularen HES-Lösungen eine starke Akkumulation im Kreislauf, während dieser Nachteil bei mittelmolekularer HES nur in abgeschwächter Form und bei niedermolekularen Präparaten praktisch nicht mehr vorhanden ist. Dass mit niedermolekularen HES-Lösungen wie mit HES 13010,4 keine Kumulation mehr auftritt, ist ein relevanter therapeutischer Fortschritt, weil sich die Plasmakonzentrationen von HES in der klinischen Routine nicht bestimmen lassen und selbst extreme Konzentrationen, zu denen es mit den hochmolekularen Lösungen binnen weniger Tage kommen kann, daher unentdeckt bleiben. Bei diesen ist die Menge der im Kreislauf kumulierenden "Rest-HES" dem Anwender unbekannt, sie beeinflusst aber gleichwohl die Kinetik und das Verhalten derjenigen HES, die in Unkenntnis der im Kreislauf noch vorhandenen Mengen additiv infundiert wurde. Hochmolekulare HES entsprechend dem Stand der Technik ist in ihrer Wirkung daher nicht berechenbar; sie bleibt länger im Kreislauf als dies aus therapeutischen Gründen in den meisten Fällen erforderlich oder erwünscht wäre und ihr metabolisches Schicksal ist ungeklärt.
Im Unterschied dazu verschwindet niedermolekulare HES innerhalb von etwa 20 bis 24 Stunden nach der Infusion vollständig aus dem Kreislauf. Dadurch werden Überhangeffekte vermieden und es kommt - speziell bei wiederholter Infusion - nicht zur Akkumulation. Das pharmakokinetische Verhalten von niedermolekularer Stärke istanders als bei hochmolekularer Stärke - berechenbar und lässt sich daher leicht steuern. Eine zu starke Belastung des Kreislaufs oder der Eliminationsmechanismen tritt nicht auf.

Dieses - für sich genommen - vorteilhafte Verhalten niedermolekularer HES im Vergleich mit hochmolekularen Präparaten wird jedoch mit einer erheblich kürzeren Plasmahalbwertszeit erkauft. Die Plasmahalbwertszeit von niedermolekularer HES beträgt nur etwa die Hälfte derjenigen von HES 200 oder weniger (J. Waitzinger et al., Clin. Drug Invest. (1998) S. 151 bis 160) und liegt im Bereich der Halbwertszeit von Gelatinepräparaten, die als ausgesprochen kurzwirksam einzustufen sind. Zwar muss eine kurze Halbwertszeit eines Volumenersatzmittels nicht grundsätzlich von Nachteil sein, da sie durch öftere bzw. höher dosierte Verabreichung des betreffenden Volumenersatzmittels kompensiert werden kann. Bei schwerer oder anhaltender Hypovolämie birgt ein Volumenersatzmittel mit kurzer Halbwertszeit und kurzer Wirkdauer jedoch die Gefahr unzureichender Kreislauffüllung (ähnlich wie mit kristalloiden Lösungen) bzw. - bei entsprechend höherer Dosierung zur Kompensation dieses Nachteils - das Risiko interstitieller Flüssigkeitsüberladung.

Vor diesem Hintergrund besteht ein Bedarf an einem Volumenersatzmittel, das sich einerseits durch geringe Kumulationsneigung und geringe Beeinflussung der Blutgerinnung auszeichnet (wie niedermolekulare HES), andererseits aber eine längere Halbwertszeit aufweist als die niedermolekularen, den kristalloiden Lösungen nahestehenden HES-Lösungen.

Auf der Suche nach einer Hydroxyethylstärke mit solchen Eigenschaften wurde jetzt gefunden, dass es sehr wohl Hydroxyethylstärke für HES-Lösungen mit längerer Plasmahalbwertszeit - im Vergleich zu bekannten niedermolekularen HES-Lösungen - gibt und diese auch hergestellt werden können und zwar überraschenderweise ohne dass diese erfindungsgemäßen hochmolekularen Lösungen die Nachteile bisheriger hochmolekularer Lösungen aufweisen, wie z. B. deren Eigenschaft, im Kreislauf zu akkumulieren, oder deren ausgeprägte Hemmung der Blutgerinnung.

Gegenstand der Erfindung in einer Ausführungsform ist daher eine Hydroxyethylstärke mit einem mittleren Molekulargewicht Mw größer oder gleich 500000, wobei der molare Substitutionsgrad MS zwischen 0,25 und 0,5, vorzugsweise von 0,35 bis 0,50 ist (0,35 ≤ MS ≤ 0,50) und das C₂/C₆-Verhältnis 2 bis unterhalb 8 beträgt.

Die erfindungsgemäßen Hydroxyethylstärken werden durch den molaren Substitutionsgrad MS beeinflusst. Der molare Substitutionsgrad MS (molar substitution) ist definiert als die durchschnittliche Anzahl von Hydroxyethylgruppen pro Anhydroglucose-Einheit (Sommermeyer et al., Krankenhauspharmazie (1987), S. 271 bis 278). Der molare Substitutionsgrad kann gemäss Ying-Che Lee et al. Anal. Chem. (1983) 55, 334 und K.L. Hodges et al., Anal. Chem (1979) 51, 2171 bestimmt werden. Eine bekannte Menge HES wird dabei unter Zusatz von Adipinsäure und lodsäure (Hl) in Xylol einer Etherspaltung unterzogen. Das freigesetzte Ethyliodid wird anschliessend gaschromatographisch unter Verwenden eines internen Standards (Toluol) und externer Standards (Ethyliodid-Eichlösungen) quantifiziert. Der molare Substitutionsgrad MS beeinflusst die Wirkung der erfindungsgemäßen Hydroxyethylstärken. Wird der MS-Wert zu hoch gewählt, so kann dies bei Einsatz der Hydroxyethylstärken als Volumenersatzmittel zu einer Kumulationswirkung im Blutkreislauf führen. Wird andererseits der MS-Wert zu niedrig gewählt, so kann dies zu einem zu raschen Abbau der Hydroxyethylstärke im Kreislauf führen und damit die gewünschte Dauer der Plasmahalbwertszeit vermindern. Als vorteilhaft hat sich ein molarer Substitutionsgrad MS von 0,35 bis 0,5 (0,35 ≤ MS ≤ 0,50), vorzugsweise 0,39 bis kleiner oder gleich 0,45 (0,39 ≤ MS ≤ 0,45) und insbesondere ein MS-Wert von größer 0,4 bis 0,44 (0,4 < MS ≤ 0,44) herausgestellt.
Die erfindungsgemäßen Hydroxyethylstärken gehören zu den höhermolekularen Hydroxyethylstärken und weisen vorzugsweise ein mittleres Molekulargewicht (Mw) oberhalb von 600000 bis 1500000, besonders bevorzugt zwischen 620000 bis 1200000 und insbesondere zwischen 700000 bis 1000000 auf. Die Hydroxyethylstärken liegen herstellungsbedingt nicht als Molekular-einheitliche Substanz mit definiertem Molekulargewicht vor, sondern als Gemisch von Molekülen unterschiedlicher Größe, die auch verschieden durch Hydroxyethylgruppen substituiert sind. Die Charakterisierung solcher Gemische bedarf daher der Zuhilfenahme statistisch gemittelter Größen. Zur Kennzeichnung des durchschnittlichen Molekulargewichts dient daher das gemittelte Molekulargewicht (Mw), wobei die allgemeine Definition dieses Mittelwertes in Sommermeyer et al., Krankenhauspharmazie (1987), S. 271 bis 278 angegeben ist.
Die Molekulargewichtsbestimmung mittels GPC-MALLS unter Verwendung der GPC-Säulen TSKgel G 6000 PW, G 5000 PW, G 3000 PW und G 2000 PW (7,5 mm x 30 cm), des MALLS-Detektors (DAWN-EOS; Wyatt Deutschland GmbH, Woldert) und des RI-Detektors (Optilab DSP; Wyatt Deutschland GmbH, Woldert) bei einer Flussrate von 1,0 ml/Minute in einem 50 mM Phosphatpuffer pH 7,0 erfolgen. Die Auswertung kann mittels ASTRA-Software (Wyatt Deutschland GmbH, Woldert) durchgeführt werden.

Bevorzugt sind solche Hydroxyethylstärken, die aus nativen oder partiell hydrolysierten Getreide- oder Kartoffelstärken erhältlich sind. Besonders vorteilhaft ist dabei aufgrund ihres hohen Gehalts an Amylopektin die Verwendung der Stärken aus Wachs-Varietäten der entsprechenden Pflanzen, sofern diese existieren (z.B. Wachsmais, Wachsreis).

Die erfindungsgemäße Hydroxyethylstärke ist ferner durch das Verhältnis der Substitution an C₂ zur Substitution an C₆ der Anhydroglucose-Einheiten beschrieben. Dieses Verhältnis, das im Rahmen dieser Erfindung auch als C₂/C₆-Verhältnis abgekürzt wird, bedeutet das Verhältnis der Zahl der in 2-Stellung substituierten Anhydroglucose-Einheiten zu der Zahl der in 6-Stellung substituierten Anhydroglucose-Einheiten der Hydroxyethylstärke. Das C₂/C₆-Verhältnis einer HES kann durch die Menge der bei der Hydroxyethylierung verwendeten Natronlauge in weiten Grenzen variiert werden, wie in Tabelle 1 und 2 dargestellt ist. Je höher die eingesetzte Menge an NaOH ist, umso stärker werden die Hydroxygruppen in 6-Stellung bei der Anhydroglucose der Stärke für die Hydroxyethylierung aktiviert. Daher nimmt das C₂/C₆-Verhältnis mit steigender NaOH-Konzentration während der Hydroxyethylierung ab. Die Bestimmung erfolgt wie von Sommermeyer et al., Krankenhauspharmazie (1987), S. 271 bis 278, angegeben. Die C₂/C₆-Verhältnisse betragen in der nachfolgenden Reihenfolge bevorzugt 3 bis unterhalb 8, 2 bis 7, 3 bis 7, 2,5 bis kleiner oder gleich 7, 2,5 bis 6 oder 4 bis 6. Das C₂/C₆-Verhältnis stellt bei den höhermolekularen HES gemäß der Erfindung einen weiteren Beitrag zur Lösung der der Erfindung zugrundeliegenden Aufgaben dar.
Die erfindungsgemäßen Hydroxyethylstärken sind aufgrund ihrer hervorragenden Verträglichkeit und ihrer guten Abbaubarkeit im menschlichen oder tierischen Organismus geeignet in den unterschiedlichsten pharmazeutischen Zubereitungen eingesetzt zu werden.
In einer besonderen Ausgestaltungsform weisen die erfindungsgemäßen HES ein mittleres Molekulargewicht von 700000 bis 1000000, einen molaren Substitutionsgrad MS von größer 0,4 bis 0,44 (0,4 < MS ≤ 0,44) und ein C₂/C₆-Verhältnis von 2 bis 7, vorzugsweise 3 bis 7 und insbesondere von 2,5 bis 6 auf.
Ein weiterer Gegenstand der vorliegenden Erfindung ist ein Verfahren zur Herstellung von Hydroxyethylstärke, vorzugsweise einer erfindungsgemäßen Hydroxyethylstärke. Vorzugsweise umfasst das Verfahren die folgenden Schritte:
(i) Umsetzung von in Wasser suspendierter Stärke, vorzugsweise Maisstärke, besonders bevorzugt partiell hydrolysierte, sog. dünnkochende Wachsmaisstärke, mit Ethylenoxid und
(ii) Teilhydrolysierung des erhaltenen Stärkederivats mit Säure, vorzugsweise Salzsäure, bis zum gewünschten mittleren Molekulargewichtsbereich der Hydroxyethylstärke.
Für die Herstellung der erfindungsgemäßen Hydroxyethylstärken eigenen sich prinzipiell alle bekannten Stärken, vor allem native oder partiell hydrolysierte Stärken, vorzugsweise Getreide- oder Kartoffelstärken, insbesondere solche mit einem hohen Gehalt an Amylopektin. In einer besonderen Ausführungsform des erfindungsgemäßen Verfahrens werden Stärken aus Wachs-Varietäten, insbesondere Wachsmais und/oder Wachsreis, eingesetzt. In einer bevorzugten Ausführungsform erfolgt die Herstellung der HES durch Umsetzung von in Wasser suspendierter Getreide- und/oder Kartoffelstärke, vorzugsweise dünnkochender Wachsmaisstärke mit Ethylenoxid. Vorteilhaft wird die Reaktion durch Zugabe von Alkalisierungsmitteln, vorzugsweise Alkalimetallhydroxiden, beispielsweise Natriumhydroxid oder Kaliumhydroxid katalysiert. In einer bevorzugten Ausführungsform des erfindungsgemäßen Verfahrens wird der in Wasser suspendierten Stärke daher zusätzlich Alkalisierungsmittel, vorzugsweise NaOH, zugesetzt. Der suspendierten Stärke wird das Alkalisierungsmittel vorteilhafterweise in einer Menge zugesetzt, so dass das molare Verhältnis von Alkalisierungsmittel zu Stärke größer als 0,2, vorzugsweise 0,25 bis 1, insbesondere 0,3 bis 0,8 ist. Durch das Verhältnis von Ethylenoxid zu Stärke während des Hydroxyethylierungsschrittes kann dabei der molare Substitutionsgrad, d.h. das molare Verhältnis von Hydroxyethylgruppen zu Anhydroglucoseeinheiten über den gewünschten MS-Bereich beliebig gesteuert werden. Bevorzugt erfolgt die Umsetzung zwischen Ethylenoxid und suspendierter Stärke in einem Temperaturbereich zwischen 30 und 70, vorzugsweise zwischen 35 und 45 °C. Üblicherweise erfolgt nach der Umsetzung die Entfernung gegebenenfalls vorhandener Restmengen des Ethylenoxids. Im Anschluss an die Umsetzung erfolgt in einem zweiten Schritt eine saure Teilhydrolyse der derivatisierten Stärke. Unter Teilhydrolyse ist dabei die Hydrolyse der alpha-glykosidisch miteinander verknüpften Glucosebausteine der Stärke zu verstehen. Prinzipiell lassen sich für die saure Hydrolyse alle dem Fachmann geläufigen Säuren einsetzen, bevorzugt sind jedoch Mineralsäuren, insbesondere Salzsäure. Die Hydrolyse kann auch enzymatisch erfolgen, wobei handelsübliche Amylasen Verwendung finden.
In einer weiteren bevorzugten Ausführungsform umfasst das erfindungsgemäße Verfahren zusätzlich die Schritte (iii) der Sterilfiltration und gegebenenfalls (iv) der Ultrafiltration. Werden in dem erfindungsgemäßen Verfahren die vorbeschriebenen Filtrationen durchgeführt, so wird die saure Teilhydrolyse der Roh-HES bis zu einem mittleren Molekulargewicht vorgenommen, das sich etwas unterhalb des gewünschten Zielmolekulargewichts befindet. Durch die Ultrafiltration können niedermolekulare Reaktionsnebenprodukte, vor allem Ethylenglykol, entfernt werden, wobei das mittlere Molekulargewicht aufgrund der Eliminierung eines Teils der niedermolekularen HES-Fraktion leicht ansteigt.
Vorzugsweise werden die aus dem Herstellverfahren hervorgehenden Lösungen anschließend auf die gewünschte HES-Konzentration verdünnt, durch Salzzusatz auf den gewünschten osmotischen Druck eingestellt, steril filtriert und in geeignete Behältnisse abgefüllt. Gegebenenfalls kann eine Sterilisation, bevorzugt durch gespannten Wasserdampf erfolgen.
Ein weiterer Gegenstand der vorliegenden Erfindung ist daher eine pharmazeutische Zubereitung enthaltend eine oder mehrere erfindungsgemäße Hydroxyethylstärke(n). Die erfindungsgemäße pharmazeutische Zubereitung kann prinzipiell in jeder erdenklichen galenischen Darbietungsform bereitgestellt werden. In einer bevorzugten Ausführungsform der vorliegenden Erfindung können die erfindungsgemäßen pharmazeutischen Zubereitungen intravenös injiziert oder infundiert werden. Bevorzugt liegen die pharmazeutischen Zubereitungen daher als wässrige Lösung oder als kolloidale wässrige Lösung vor. Vorzugsweise enthalten die Zubereitungen die erfindungsgemäßen Hydroxyethylstärken in einer Konzentration von bis zu 20, weiter bevorzugt von 0,5 bis 15, besonders bevorzugt von 2 bis 12, insbesondere von 4 bis 10, beispielsweise 6 %.
Die Mengenangaben erfolgen, wenn nicht anders angegeben in %, das im Rahmen der vorliegenden Erfindung gleichzusetzen ist mit g/100 ml Lösung.

In einer weiteren Ausführungsform enthalten die erfindungsgemäßen pharmazeutischen Zubereitungen zusätzlich Natriumchlorid, vorzugsweise 0,6 bis 2 %, besonders bevorzugt 0,9 %. Eine 0,9 prozentige Lösung von Natriumchlorid in Wasser wird auch als physiologische Kochsalzlösung bezeichnet. Sie weist den gleichen osmotischen Druck wie das Blutserum auf und ist daher als isotonische Lösung zur intravenösen Injektion oder Infusion geeignet. Zur Isotonisierung können auch alle anderen osmotisch wirksamen Substanzen verwendet werden, soweit sie physiologisch unbedenklich und gut verträglich sind wie z. B. Glucose, Glucoseersatzstoffe (Fructose, Sorbit, Xylit) oder Glycerin. In einer weiteren bevorzugten Ausführungsform können die pharmazeutischen Zubereitungen zusätzlich weitere plasmaadaptierte Elektrolyte enthalten. Die Herstellung solcher isotoner Zubereitungen ist dem Fachmann bekannt. Ein Beispiel für eine isotone Lösung mit plasmaadaptierten Elektrolyten ist die sogenannte Tyrode-Lösung. Sie enthält 0,8 g NaCl, 0,02 g KCI, 0,02 g CaCl₂, 0,01 g MgCl₂, 0,005 g NaH₂PO₄, 0,1 g NaHCO₃ und 0,1 g Glucose in 100 ml destilliertem Wasser. Ein weiteres Beispiel ist die sogenannte Ringer-Lösung, die 0,8 % Natriumchlorid, 0,02 % Kaliumchlorid, 0,02 % Calciumchlorid und 0,1 % Natriumhydrogencarbonat aufweist. Hierbei können selbstverständlich auch die Anionen der Elektrolyte durch metabolisierbare Anionen ausgetauscht werden, so kann beispielsweise in der Ringer-Lösung das Natriumhydrogencarbonat durch 0,3 oder 0,6 % Natriumlactat ersetzt werden. Eine entsprechende Elektrolytzusammensetzung bzw. -lösung ist dem Fachmann als "Ringer-Lactat" bekannt. Weitere metabolisierbare Anionen, die allein oder in Kombination Verwendung finden können, sind Acetat (z.B. "Ringer-Acetat") oder Malat.
In einer weiteren Ausführungsform der Erfindung können die pharmazeutischen Zubereitungen auch als hypertone Lösungen vorliegen. Hypertone Lösungen sind solche mit einem höheren osmotischen Druck als das menschliche Blut. Die Applikation hypertoner pharmazeutischer Zubereitungen kann bei bestimmten Krankheitsbildern vorteilhaft sein. Der erforderliche hohe osmotische Druck hypertoner Lösungen wird durch Zugabe entsprechender Mengen osmotisch wirksamer Substanzen eingestellt, z.B. durch Natriumchlorid, das zu diesem Zweck in Konzentrationen bis 7,5 % und mehr verwendet werden kann.

Zur Vermeidung und Reduzierung der Gefahr von Infektionen werden die erfindungsgemäßen pharmazeutischen Zubereitungen vorzugsweise sterilfiltriert oder hitzesterilisiert. Zwecks Sterilfiltration erfindungsgemäßer wässriger oder kolloidaler wässriger pharmazeutischer Zubereitungen eignen sich insbesondere feinporige Filterkartuschen, wie sie beispielsweise unter dem Markennamen SARTPORE von der Firma Sartorius zur Verfügung gestellt werden. Geeignet sind beispielsweise solche Filterkartuschen mit einem Porendurchmesser von 0,2 µm. Die erfindungsgemäßen pharmazeutischen Zubereitungen können darüber hinaus hitzesterilisiert werden, ohne dass es zu einer Beeinträchtigung der Hydroxyethylstärken kommt. Vorzugsweise wird die Hitzesterilisation bei einer Temperatur oberhalb von 100 °C, besonders bevorzugt zwischen 105 und 150 °C, insbesondere zwischen 110 und 130 °C, beispielsweise 121 °C über einen Zeitraum von bis zu 30 Minuten, vorzugsweise bis zu 25 Minuten, insbesondere zwischen 18 und 22 Minuten durchgeführt.
In einer bevorzugten Ausführungsform ist die pharmazeutische Zubereitung ein Volumenersatzmittel. Volumenersatzmittel finden ihren Einsatz beim Ersatz intravasaler Flüssigkeit in tierischen und menschlichen Organismen. Volumenersatzmittel finden insbesondere bei der Prophylaxe und Therapie der Hypovolämie ihren Einsatz. Es ist dabei unbedeutend, ob die Hypovolämie aus dem unmittelbaren Verlust von Blut bzw. Körperflüssigkeiten resultiert, wie beispielsweise bei akuten Blutungen, Traumen, Operationen, Verbrennungen, usw. oder aus Verteilungsstörungen zwischen Makro- und Mikrozirkulation, wie beispielsweise bei der Sepsis oder aus einer Vasodilatation, wie beispielsweise bei der Einleitung der Anästhesie. Die Volumenersatzmittel werden dabei weiter unterteilt in die sogenannten Plasmaersatzmittel und die sogenannten Plasmaexpander. Bei den Plasmaersatzmitteln entspricht das intravasal applizierte Volumen des Mittels auch dem Volumen, das den Gefäßen zugeführt wird. Bei den Plasmaexpandern hingegen ist das intravasal applizierte Flüssigkeitsvolumen des Expanders geringer als das den Gefäßen real zugeführte Volumen. Dieses Phänomen liegt darin begründet, dass durch den Einsatz von Plasmaexpandern das onkotische Gleichgewicht zwischen Intra- und Extravasalraum gestört wird und zusätzliches Flüssigkeitsvolumen in das zu behandelnde Gefäßsystem aus dem extravasalen Raum einströmt.

Plasmaexpander unterscheiden sich von den Plasmaersatzmitteln im Wesentlichen dadurch, dass die Konzentration der enthaltenen erfindungsgemäßen Hydroxyethylstärken erhöht ist und/oder die Konzentration der jeweiligen Elektrolyte ein onkotisches und/oder osmotisches Ungleichgewicht hervorrufen.
Die erfindungsgemäße pharmazeutische Zubereitung kann ferner einen pharmazeutischen Wirkstoff oder Wirkstoffkombinationen enthalten und so als Medium zur Verabreichung der darin gelösten Wirkstoffe, insbesondere durch Injektion und Infusion dienen.
Ein weiterer Gegenstand der vorliegenden Erfindung ist die Verwendung einer erfindungsgemäßen pharmazeutischen Zubereitung zur Herstellung eines Volumenersatzmittels oder eines Plasmaersatzmittels oder eines Plasmaexpanders.
Die erfindungsgemäßen pharmazeutischen Zubereitungen können besonders bevorzugt als Volumenersatzmittel oder Plasmaersatzmittel oder Plasmaexpander verwendet werden. Bevorzugt dienen die pharmazeutischen Zubereitungen der Aufrechterhaltung der Normovolämie. Die Auftrechterhaltung der Normovolämie ist von besonderer Bedeutung für die hämodynamische Stabilität, die einen entscheidenden Einfluss auf den menschlichen oder tierischen Organismus, beispielsweise bezüglich des Blutdrucks, der Diureserate oder der Herzfrequenz hat. Um die Aufrechterhaltung der Normovolämie möglichst schnell nach einem intravasalen Flüssigkeitsverlust zu kompensieren, haben sich die erfindungsgemäßen pharmazeutischen Zubereitungen als besonders vorteilhaft erwiesen, da sie im Vergleich zu den im Stand der Technik bekannten Plasmaersatzmitteln, speziell niedermolekularen HES-Lösungen, wie z.B. HES 130/0,4, eine verlängerte Plasmahalbwertszeit aufweisen, insbesondere in der entscheidenden Phase unmittelbar nach der Infusion. Vorteilhaft sind die erfindungsgemäßen pharmazeutischen Zubereitungen insbesondere auch deshalb, da überraschenderweise gefunden wurde, dass bei Verwendung der Zusammensetzungen die Blut- und/oder Plasmaviskosität im Unterschied zu der in US 5,502,043 für hochmolekulare HES getroffenen Aussage nicht ansteigt und da die Blutgerinnung weniger gehemmt wird als mit anderen hochmolekularen Zubereitungen. Dass die Plasmaviskosität überraschenderweise nicht ansteigt, sorgt dabei ebenfalls für eine Verbesserung der Mikrozirkulation sowie zu einer verbesserten nutritiven Sauerstoffversorgung der Gewebe.

Ein weiterer Gegenstand ist die Verwendung der erfindungsgemäßen pharmazeutischen Zubereitung zur Aufrechterhaltung der Normovolämie und/oder zur Verbesserung der Makro- und Mikrozirkulation und/oder zur Verbesserung der nutritiven Sauerstoffversorgung und/oder zur Stabilisierung der Hämodynamik und/oder zur Verbesserung der Volumenwirksamkeit und/oder zur Verringerung der Plasmaviskosität und/oder zur Erhöhung der Anämietoleranz und/oder zur Hämodilution, insbesondere zur therapeutischen Hämodilution bei Durchblutungsstörungen und arteriellen, speziell peripheren arteriellen, Verschlusskrankheiten.

Die erfindungsgemäßen pharmazeutischen Zubereitungen oder die erfindungsgemäße Hydroxyethylstärke werden vorzugsweise zur Herstellung von Medikamenten, insbesondere für Medikamente zur Aufrechterhaltung der Normovolämie und/oder zur Verbesserung der Makro- und Mikrozirkulation und/oder zur Verbesserung der nutritiven Sauerstoffversorgung und/oder zur Stabilisierung der Hämodynamik und/oder zur Verbesserung der Volumenwirksamkeit und/oder zur Verringerung der Plasmaviskosität und/oder zur Erhöhung der Anämietoleranz und/oder zur Hämodilution, insbesondere zur therapeutischen Hämodilution bei Durchblutungsstörungen und arteriellen, speziell peripheren arteriellen, Verschlusskrankheiten verwendet.

Darüber hinaus werden die erfindungsgemäßen pharmazeutischen Zubereitungen oder die erfindungsgemäßen Hydroxyethylstärken vorteilhafterweise in Verfahren zur Behandlung der Aufrechterhaltung der Normovolämie und/oder Verbesserung der Makro- und Mikrozirkulation und/oder Verbesserung der nutritiven Sauerstoffversorgung und/oder Stabilisierung der Hämodynamik und/oder Verbesserung der Volumenwirksamkeit und/oder Verringerung der Plasmaviskosität und/oder Erhöhung der Anämietoleranz und/oder Hämodilution, insbesondere therapeutischen Hämodilution bei Durchblutungsstörungen und arteriellen, speziell peripheren arteriellen, Verschlusskrankheiten eingesetzt.

Ein weiterer Gegenstand der vorliegenden Erfindung ist ein Kit, umfassend in getrennter Form
(i) eine erfindungsgemäße Hydroxyethylstärke,
(ii) eine sterile Salz-Lösung, vorzugsweise Natriumchlorid-Lösung, und gegebenenfalls
(iii) einen pharmazeutischen Wirkstoff oder eine Wirkstoffkombination.

In einer bevorzugten Ausführungsform weist das erfindungsgemäße Kit die Einzelkomponenten (i), (ii) und gegebenenfalls (iii) in getrennten Kammern in einem Mehrkammerbeutel auf, wobei sowohl alle Komponenten getrennt, wie auch bestimmte Komponenten, z.B. (i) und (ii), in einer Kammer gemeinsam vorliegen können.

Die Erfindung wird durch die nachstehenden Beispiele näher erläutert.

### Herstellungsbeispiele für HES-Rohstoffe

### Beispiel 1: Herstellung von HES-Rohstoffen mit identischem MS und C₂/C₆-Verhältnis, aber unterschiedlichen Molekulargewichten

Die im experimentellen Teil beschriebenen HES-Spezies für die in vivo-Untersuchungen wurden aus einem einzigen Reaktionsansatz durch fraktionierte Hydrolyse hergestellt. Zu diesem Zweck wurde wie folgt verfahren. 30 kg dünnkochende Wachsmaisstärke wurden in 52.2 kg WFI (Wasser für Injektionszwecke) bei Raumtemperatur unter starkem Rühren suspendiert. Um die Stärke optimal zu hydratisieren, wurde die Suspension anschließend durch Erhitzen auf mindestens 85°C verkleistert. Nach mehrmaliger Inertisierung der Suspension mit Stickstoff mittels 10 minütiger Durchleitung von Stickstoff und nachfolgender Evakuierung mittels Vakuum wurde die Stärke durch Zugabe von 5,1 kg NaOH aktiviert. Anschließend wurde 4,159 kg gekühltes Ethylenoxid in flüssiger Form in den Reaktor geleitet und die Temperatur langsam auf 40 °C erhöht und die Reaktionsmischung 2 Stunden unter fortgesetztem Rühren bei dieser Temperatur belassen. Nicht umgesetztes Ethylenoxid wurde durch mehrmaliges Inertisieren wie oben beschrieben aus dem Reaktionsansatz entfernt. Aus dieser Roh-HES wurden dann durch schrittweise saure Hydrolyse 3 HES-Präparate mit identischem Mw und C₂/C₆-Verhältnis, aber unterschiedlichem Mw hergestellt. Zur Reduktion des Molekulargewichtes wurde die Lösung mit 20 %iger HCl auf pH 2,0 eingestellt, auf 75 ± 1 °C aufgeheizt und solange bei dieser Temperatur belassen, bis das mittlere Molekulargewicht Mw des HES-Kolloids, bestimmt mittels GPC-MALLS, auf 865 kD abgesunken war. Ein Drittel des Hydrolyseansatzes wurde dem Reaktor entnommen und sofort auf eine Temperatur von unter 50 °C abgekühlt. Nach Entfärbung der Lösung durch Aktivkohlebehandlung wurde die Lösung mittels handelsüblicher Vorfilter und Sterilfilter filtriert und nach Verdünnung auf 12 % durch Ultrafiltration (UF) aufgereinigt. Dazu wurden Polyethersulfon-Membranen der Firma Millipore mit einem cut-off von 10 kD verwendet. Im Lauf der UF steigt das Mw aufgrund der teilweisen Elimination der niedermolekularen HES Fraktion etwas an. Dieser Anstieg ist abhängig vom Ausgangs-Mw der Kolloidpräparation, vor allem aber vom deklarierten cut-off der eingesetzten UF-Membran und der eingesetzten UF-Membran-Charge. Um ein gewünschtes Zielmolekulargewicht nach UF zu erzielen, muss der Mw-Shift während der UF vorab mit der vorhandenen UF-Membran-Charge experimentell ermittelt werden. Zu beachten ist ebenfalls, dass vom Zeitpunkt der Probenentnahme zur Bestimmung des Mw während der sauren Hydrolyse bis zum Vorliegen des ermittelten Mw-Wertes die Hydrolyse weiter voranschreitet. Daher ist die Abnahme des Mw über die gesamte Hydrolysedauer systematisch zu erfassen und durch Extrapolation des Mw über die Zeit der Zeitpunkt abzuschätzen, bei dem das Ziel-Mw erreicht sein wird. Zu diesem extrapolierten Zeitpunkt wird die Hydrolyse dann abgebrochen. Der nach Entnahme des ersten Drittels verbliebene Hydrolyseansatz wurde in der Zwischenzeit so lange weitergefahren, bis das mittlere Molekulargewicht Mw auf 460 kD abgesunken war. Anschließend wurde das zweite Drittel genauso aufgearbeitet wie das erste Drittel. Das verbleibende Drittel wurde parallel dazu bis zu einem Mw von 95 kD weiterhydrolysiert und dem gleichen Aufarbeitungsverfahren unterzogen wie die beiden anderen Teilansätze. Aus Teilansatz 1 war HES 900/0,42 (C₂/C₆-Verhältnis = 4,83), aus Teilansatz 2 HES 500/0,42 (C₂/C₆-Verhältnis = 4,83) und aus Teilansatz 3 HES 130/0,42 (C₂/C₆-Verhältnis = 4,83) zugänglich.

Nach Beendigung der Ultrafiltration wurde die Kolloidkonzentration auf 6 % und der pH-Wert auf 5,5 eingestellt, die Lösung durch Zusatz von NaCl isotonisiert, in Glasflaschen à 500 ml abgefüllt und 20 Minuten bei 121°C sterilisiert.

### Beispiel 2: Herstellung weiterer HES-Rohstoffe

Um HES-Kolloide mit anderen molaren Substitutionsgraden und C₂/C₆-Verhältnissen zu erzeugen, wurde eine Reihe weiterer Experimente in der gleichen Ansatzgrösse durchgeführt, wobei die Menge an Ethylenoxid entsprechend variiert wurde. Ausserdem wurde die saure Hydrolyse beim Erreichen unterschiedlicher Mw (Ziel-Mw) abgestoppt Diese Experimente sind in der nachfolgenden Tabelle 1 zusammenfassend dargestellt:

**Tabelle 1**

| | Bsp.1 | Bsp. 2 | Bsp. 3 | Bsp. 4 | Bsp. 5 |
|---|---|---|---|---|---|
| **Reaktionsbedingungen** | | | | | |
| Stärke (kg) | 30,0 | 30,0 | 30,0 | 30,0 | 30,0 |
| WFI (kg) | 52,0 | 52,0 | 52,0 | 52,0 | 52,0 |
| NaOH 50 % (kg) | 9,5 | 4,7 | 4,7 | 9,5 | 4,7 |
| Ethylenoxid (kg) | 4,0 | 3,6 | 4,4 | 4,5 | 3,8 |
| Ziel-Mw (nach saurer Hydrolyse) (kDa) | 1500 | 990 | 885 | 770 | 665 |

| **HES-Charakteristik** | | | | | |
|---|---|---|---|---|---|
| MS (mol/mol) | 0,39 | 0,40 | 0,45 | 0,46 | 0,42 |
| Mw nach UF (kDa) | 1520 | 1050 | 915 | 795 | 710 |
| C₂:C₆-Verhältnis | 2,3 | 6,0 | 5,9 | 2,2 | 6,1 |

### Beispiel 3: Einfluss des molaren Verhältnisses von NaOH zu Stärke während der Hydroxyethylierung auf das C₂/C₆-Verhältnis

Um die Steuerbarkeit des C₂/C₆-Verhältnisses durch das molare Verhältnis von NaOH zu Anhydroglucoseeinheiten der Stärke aufzuzeigen, wurden 30 kg dünnkochende Wachsmaisstärke mit unterschiedlichen Mengen NaOH versetzt und mit Ethylenoxid bei 40°C umgesetzt. In Tabelle 2 sind die eingesetzten Mengen an Reagenzien und die C₂/C₆-Verhältnisse sowie das MS der durch diese Reaktion gewonnenen HES-Produkte aufgeführt. Wie zu erkennen, nimmt das C₂/C₆-Verhältnis mit steigendem Verhältnis NaOH vs. Stärke ab. Dies ist darauf zurückzuführen, dass die basenkatalysierte Hydroxyethylierung der Stärke bei niedriger NaOH-Konzentration bevorzugt an den Hydroxyethylgruppen in 2-Position der Anhydroglucoseeinheiten erfolgt, die am reaktivsten sind. Durch höhere NaOH-Konzentrationen werden auch die per se weniger reaktiven C₆-Hydroxygruppen genügend stark aktiviert, um effizient hydroxyethyliert zu werden.

**Tabelle 2: Steuerung des C₂/C₆-Verhältnisses während der Hydroxyethylierung**

| Versuch | WFI [kg] | Stärke [kg] | NaOH (50 Gew.-%) [kg] | Molares Verhältnis NaOH vs. Stärke | Ethylenoxid [kg] | MS | C₂/C₆ |
|---|---|---|---|---|---|---|---|
| # 1 | 50 | 30 | 1,5 | 0,1 | 4,0 | 0,4 | 12 |
| # 2 | 50 | 30 | 4,5 | 0,3 | 3,8 | 0,4 | 7 |
| # 3 | 50 | 30 | 11,1 | 0,75 | 4,4 | ,04 | 3 |

### Herstellungsbeispiele für HES-Fertigprodukte

In der nachfolgenden Tabelle 3 sind die Rezepturen für die Herstellung verschiedener HES-Lösungen aufgeführt. Die HES wurde als HES-Konzentrat nach der Ultrafiltration eingesetzt. Die Menge an HES-Konzentrat, die zur Herstellung einer 6%igen bzw. 10%igen HES Lösung benötigt wurde, wurde dabei durch Dreisatzrechnung bestimmt. Eine andere Möglichkeit besteht darin sprühgetrocknete HES zu verwenden, was für den Fachmann, der mit einem Sprühturm ausgerüstet ist, keine Schwierigkeit darstellt. Die verwendete HES hatte ein Molekulargewicht von 900 kD und ein MS von 0,42.

In einen 200 I Ansatztank wurden jeweils die benötigte Menge an HES-Konzentrat und die in der Tabelle angegebenen Mengen der Salze und NaOH-Lösung eingewogen und die Salze unter Rühren gelöst. Nach pH-Einstellung der Lösungen 1, 4, 5, 7 und 8 auf 5,5 und der Lösungen 2, 3, 6 auf 6,0 wurde soviel Wasser für Injektionszwecke (WFI) zugegeben, dass die theoretischen Na-Konzentrationen gemäss Spezifikation erreicht wurden.

Wie es dem Fachmann ohne weiteres ersichtlich ist, können durch Änderung der Einwaage der aufgeführten wirksamen Bestandteile bzw. Hilfsstoffe sowie durch das Weglassen oder Hinzufügen weiterer Substanzen die Rezepturen in weiten Grenzen variiert und bei Verwendung anderer HES-Spezies auch mit diesen entsprechende Lösungen hergestellt werden.

**Tabelle 3**

| | 1 | 2 | 3 | 4 | 5 | 6 | 7 | 8 |
|---|---|---|---|---|---|---|---|---|
| **Herstellungsrezepturen** | | | | | | | | |
| HES [g/l] | 60 | 60 | 60 | 60 | 100 | 100 | 100 | 100 |
| NaCl [g/l] | 9,00 | 6,369 | 17,37 | 100,0 | 9,00 | 6,369 | 30,00 | 60,00 |
| KCI [g/l] | 0 | 0,373 | 0,373 | 0 | 0 | 0.373 | 0 | 0 |
| MgCl₂ [g/l] | 0 | 0,203 | 0,203 | 0 | 0 | 0.203 | 0 | 0 |
| CaCl₂ [g/l] | 0 | 0,294 | 0,294 | 0 | 0 | 0.294 | 0 | 0 |
| Na-Acetat [g/l] | 0 | 5,032 | 5,032 | 0 | 0 | 5.032 | 0 | 0 |

| **Analysenergebnisse der Lösungen** | | | | | | | | |
|---|---|---|---|---|---|---|---|---|
| HES [%] | 6,0 | 6,1 | 6,0 | 5,9 | 10,0 | 10,1 | 9,9 | 10,0 |
| Na [mM] | 153,9 | 146,1 | 334,3 | 1711 | 153,7 | 146,0 | 512,5 | 1026 |
| K [mM] | 0 | 5,1 | 5,0 | 0 | 0 | 5,0 | 0 | 0 |
| Mg [mM] | 0 | 1,0 | 0,9 | 0 | 0 | 1,0 | 0 | 0 |
| Ca [mM] | 0 | 2,1 | 2,0 | 0 | 0 | 2,0 | 0 | 0 |
| Cl [mM] | 154,8 | 120,2 | 308,5 | 1712 | 154,8 | 120,1 | 513,7 | 1028 |
| Acetat [mM] | 0 | 37,0 | 37,0 | 0 | 0 | 37,0 | 0 | 0 |
| pH | 5.5 | 6,0 | 5,9 | 5,3 | 5,4 | 6,1 | 5,4 | 5,5 |

### Messmethoden der Anwendungsbeispiele

Im Folgenden werden die Meßmethoden beschrieben, mit denen die Blut- und Plasmaproben untersucht wurden.

### Nativblutmessungen:

Mit Citrat versetzte Blutproben wurden im Labor wie folgt behandelt:
Eine Probe wurde sofort für die Blutviskosiätsmessung verwendet (Rheostress^{®} 1, Thermo-Haake, Karlsruhe, Deutschland) bei linear ansteigenden Scherraten von 1 - 240 pro Sekunde. Die Viskosität wurde bei Scherraten von 1 pro Sekunde und 128 pro Sekunde untersucht. Vor der Thromboelastograph^{®} Analyse (TEG^{®}, Haemoscope Corporation, Niles, Illinois) wurden die Blutproben für eine Stunde in einem 37 °C warmen Wasserbad inkubiert. Die Blutrekalzifizierung und die TEG^{®}-Messungen wurden entsprechend der Herstelleranleitung durchgeführt. Bestimmt wurde der Koagulationsindex (Cl), der mehrere Partialfunktonen der Thrombelastographie zusammenfast.

### Plasma-Messungen:

Die Blutproben wurden für 15 Minuten bei 4 °C und 3000 U/min. zentrifugiert (Rotana/RP, Hettich, Bäch, Schweiz). Die Plasmaviskosität wurde entsprechend, wie bereits für die Blutbestimmung beschrieben, gemessen.

Die Prothrombin-Zeit (PT) und die aktivierte partielle Thromboplastin-Zeit (aPTT) wurden mittels eines automatischen Koagulations-Analyzers (BCS, Dade Behring, Marburg, Deutschland) mit Hilfe eines PT-Reagenzes, enthaltend einen rekombinanten Gewebefaktor (Innovin^{®}, Dade Behring) und einem aPTT-Reagenz, enthaltend Ellagsäure (Actin FS^{®}, Dade Behring) bestimmt. PT-Werte wurden in INR-Werte, basierend auf den ISI-Werten, die vom Hersteller geliefert wurden, überführt. Die funktionelle Aktivität des von-Willebrand-Faktors (vWF) wurde bestimmt mittels eines kommerziellen Ristocetin-Cofaktor-Assays (vWF RCA, Dade Behring) in einem automatisierten Koagulations-Analyzer (BCS, Dade Behring). Die vWF-Aktivität wurde ermittelt anhand der Fähigkeit menschliche Thrombozyten in Gegenwart von Ristocetin zu agglutinieren. Die Agglutination wurde anhand von Trübheitsmessungen durch den Koagulations-Analyzer bestimmt. Antigen-vWF wurde nachgewiesen durch ein kommerzielles ELISA-Kit (Asserachrom vWF antigenic, Roche Diagnostics, Rotkreuz, Schweiz) entsprechend der Hersteller-Anleitung.

Die HES-Konzentration wurde nach Extraktion aus dem Blutplasma und Hydrolyse zu Glucosemonomer-Einheiten quantifiziert (H. Förster et al., lnfusionstherapie 1981; 2: 88-94). Die Plasma-Proben (1 ml) wurden bei 100 °C für 60 Minuten inkubiert, nachdem ihnen 0,5 ml KOH-Lösung 35 %ig (w/w) (Fluka, Buchs, Schweiz) zugegeben wurde. Die HES wurde durch Zugabe von 10 ml eiskaltem absoluten Ethanol (Fluka, Buchs, Schweiz) zum Überstand der Reaktionsmischung ausgefällt und anschließend durch Säurehydrolyse in 2 N HCl (Fluka, Buchs, Schweiz) für 60 Minuten bei 100°C hydrolisiert. Die Glucosebestimmung erfolgte durch Einsatz eines Enzym-Testkits basierend auf Hexokinase/Glucose 6-phosphatase (Boehringer Mannheim, Darmstadt, Deutschland).

Die Berechnung der pharmakokinetischen Parameter erfolgte unter Annahme eines Zwei-Kompartiment-Modells mit konstanter Infusionsrate unter Verwendung der tat sächlichen Dosierungen und Infusionsdauern (WinNonlin, Version 4.1, Pharsight Corp, Mountainview, CA).

### Statistische Analyse:

Die Werte werden als Mittelwert ± Standardabweichung angegeben. Die beiden HES-Lösungen mit hohem Molekulargewicht (500 und 900 kD) wurden verglichen mit der niedrigmolekularen (130 kD) Lösung mittels des JMP 5.1 Statistikpakets (SAS Institute, Inc, Cary, NC). Die Wechselwirkung von Lösungs- und Zeiteffekten wurde mittels zweiseitiger ANOVA-Analyse und unter Berücksichtigung der Korrektur nach Bonferroni geprüft. Für die statistische Analyse der pharmakokinetischen Parameter wurde der t-Test für ungepaarte Daten nach Student verwendet. Ein p < 0,05 wurde als statistisch signifikant gewertet.

### Anwendungsbeispiele

Für die nachstehend beschriebenen in vivo-Experimente wurden erfindungsgemäße Hydroxyethylstärken mit mittleren Molekulargewichten (Mw) von 500000 und 900000 Dalton und identischer molarer Substitution (MS = 0,42) und identischem C₂/C₆-Verhältnis (4.83) (nachfolgend in den Anwendungsbeispielen als HES 500/0,42 bzw. HES 900/0,42 bezeichnet) verwendet (siehe Herstellungsbeispiel für HES-Rohstoffe). Beide Hydroxyethylstärken (HES 900/0,42 und HES 500/0,42) wurden in 0,9 %iger Kochsalzlösung in einer Konzentration von 6 % aufgelöst, unter Verwendung von 0,2 µm Filterkartuschen (Sartpore; Sartorius), sterilfiltriert, in Glasflaschen abgefüllt und 15 Minuten bei 121 °C hitzesterilisiert. Als Vergleichslösung diente eine niedermolekulare Hydroxyethylstärke (Mw = 130000 Dalton) mit identischem MS und C₂/C₆-Verhältnis (nachfolgend in den Anwendungsbeispielen als HES 130/0,42 bezeichnet), die ebenfalls in 6 %iger Konzentration in 0,9 % Kochsalzlösung vorlag. Wie beschrieben wurde sie aus dem gleichen Reaktionsansatz wie die erfindungsgemäßen hochmolekularen Stärken erhalten, von denen sie sich infolgedessen nur durch das Molekulargewicht unterschied.

### Untersuchung der Plasmaelimination und des Einflusses auf die Blutgerinnung

30 Schweine wurden in 3 Kohorten zu jeweils 10 Tieren randomisiert. Die eine Kohorte erhielt eine intravenöse Infusion von HES 900/0,42, die andere eine Infusion von HES 500/0,42 und die dritte eine Infusion von HES 13010,42 als Vergleich. In allen Fällen betrug die Dosis 20 ml/kg KG der jeweils 6 %igen HES-Lösungen, und die Infusion dauerte 30 Minuten. Für die Infusion und die nachfolgenden Blutentnahmen waren die Tiere anästhesiert (Halothan-Narkose) und kontrolliert beatmet. Blutentnahmen erfolgten vor Infusionsbeginn, nach 5, 20, 40, 60, 120 und 240 Minuten sowie 24 Stunden nach Infusionsende. In den Blut- bzw. den daraus gewonnenen Plasmaproben wurden bestimmt: Blut- und Plasmaviskosität, HES-Konzentration, Prothrombinzeit, partielle Thromboplastinzeit, von Willebrand Faktor, Faktor VIII und Ristocetin-Kofaktor sowie die üblichen thrombelastographischen Kenngrößen. Aus dem Verlauf der HES-Konzentrationen von Infusionsende bis 24 h danach wurden die Fläche unter der Konzentrationszeit-Kurve (= AUC von Area Under the Curve), die α- und β-Halbwertszeit sowie die Clearance berechnet. Die Berechnung der AUC erfolgte nach der log-linearen Trapezregel, und für die Berechnung der übrigen pharmakokinetischen Parameter wurde ein 2-Kompartiment-Modell zugrundegelegt. Aus diesem ergeben sich 2 Halbwertszeiten α und β, wobei die α-Halbwertszeit den Übertritt des HES vom zentralen Kompartiment (entspricht weitgehend dem Intravasalraum) in das periphere Kompartiment kennzeichnet und die β-Halbwertszeit die Rückverteilung in umgekehrter Richtung.

Der Verlauf der HES-Konzentration und die pharmakokinetischen Parameter ließen eine längere Plasmaverweildauer der hochmolekularen Varianten (HES 900/0,42 und HES 500/0,42) im Vergleich zu der niedermolekularen HES (HES 130/0,42) erkennen. So waren AUCs und die α-Halbwertszeiten bei den hochmolekularen Varianten signifikant größer bzw. länger als bei der niedermolekularen Kontrolle; dementsprechend war die Clearance der hochmolekularen HES-Typen signifikant niedriger als die der niedermolekularen HES.

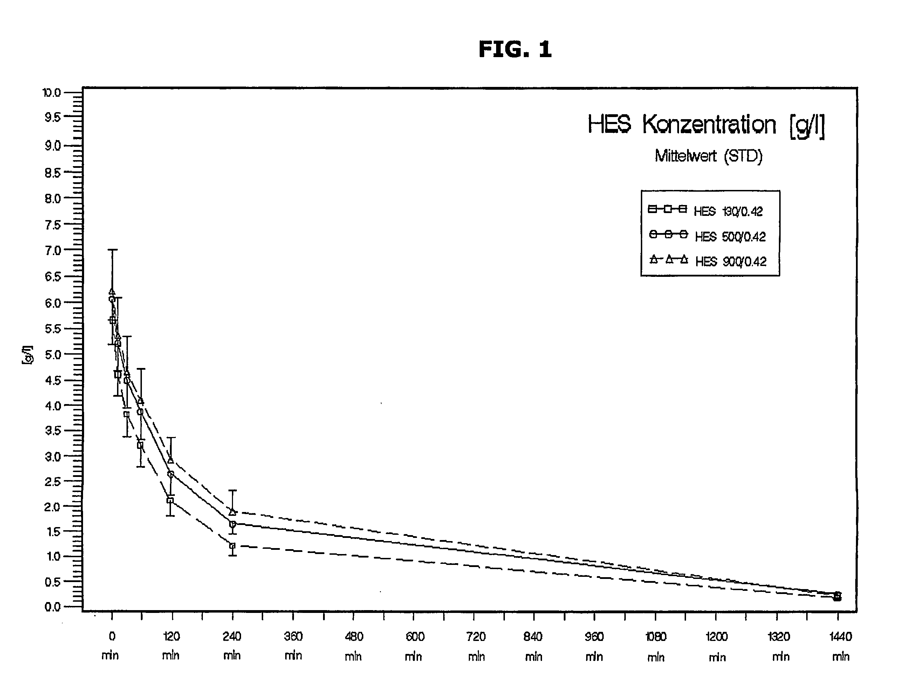

Überraschenderweise und völlig anders als bei bisher bekannten Typen von mittel- oder hochmolekularer HES (HES 200/0,5; HES 200/0,6; HES 450/0,7) bestand jedoch bei der Plasmakonzentration zum Zeitpunkt "24 Stunden nach Infusion" kein relevanter Unterschied zwischen den erfindungsgemäßen hochmolekularen Varianten und der niedermolekularen Vergleichslösung (vgl. Figur 1). Dies bedeutet, dass die erfindungsgemäßen hochmolekularen Hydroxyethylstärken in der für die Volumenwirksamkeit entscheidenden Phase unmittelbar nach der Infusion eine signifikant längere Plasmaverweildauer besitzen als die niedermolekulare Vergleichs-HES, dass sie aber anders als bisher bekannte hochmolekulare HES-Typen keine Neigung zur Kumulation im Kreislauf zeigen. Stattdessen waren die erfindungsgemäßen HES-Varianten 24 Stunden nach der Infusion ebenso wie die niedermolekulare Vergleichs-HES praktisch vollständig aus dem Kreislauf verschwunden.

**Tabelle 4**

| **Lösung** | **HES 130/0,42** | **HES 500/0,42** | **HES 900/0,42** |
|---|---|---|---|
| AUC (g*min/l) | 1156 ± 223 | 1542 ± 142** | 1701 ± 321** |
| CL (ml/min) | 39,1 ± 7,9 | 30,1 ± 5,4* | 26,0 ± 4,1** |
| t_{1/2α} (min) | 39,9 ± 10,7 | 53,8 ± 8,6* | 57,1 ± 12,3* |
| t_{½β} (min) | 331,8 ± 100,0 | 380,6 ± 63,3 | 379,9 ± 75,8 |

| | | | |
|---|---|---|---|
| Tab. 4: Fläche unter der Konzentration-Zeit-Kurve (AUC), Clearance (CL), alpha- und beta-Halbwertszeit (t_{½α} und t_{½β}) nach Infusion von jeweils 20 ml/kg 6 % HES 130/0,42, 6 % HES 500/0.42 bzw. 6 % HES 900/0,42 beim Schwein. Die Signifikanzprüfung erfolgte zwischen HES 500 und HES 900 jeweils im Vergleich zu HES 130/0,42 mittels t-Test für ungepaarte Daten nach Student * p < 0.01; ** p < 0.001. Die hochmolekularen HES-Spezies (HES 500/0,42 und HES 900/0,42) zeigten signifikant größere Flächen unter der Konzentrations-Zeit-Kurve (AUC) entsprechend einer höheren Verweildauer im Intravasalraum, signifikant längere initiale Plasmahalbwertszeiten (t_{½α}) und signifikant niedrigere Clearanceraten als niedermolekulare (HES 130/0,42). | | | |

Figur 1 zeigt den Konzentrationsverlauf von niedermolekularer HES (HES 130/0,42) und hochmolekularer HES (HES 500/0,42 und HES 900/0,42) im Plasma nach Infusion von 20 ml/kg der jeweiligen HES-Lösung beim Schwein. HES 500/0,42 und HES 900/0,42 wurden anfangs langsamer aus dem Intravasalraum eliminiert als HES 130/0,42 (s.a. Tabelle 4: Pharmakokinetische Parameter); in der Endphase der Elimination, d.h. 24 h nach Infusionsende, bestand jedoch kein relevanter Unterschied zwischen den Plasmakonzentrationen mehr (die mittleren Konzentrationen lagen unter 0.2 g/l und damit im Bereich der Bestimmungsgrenze).

Es hat sich somit gezeigt, dass die erfindungsgemäßen Hydroxyethylstärken einerseits eine längere initiale Plasmahalbwertszeit besitzen als zur Zeit bekannte niedermolekulare Referenzlösungen (HES 130/0,42), aber andererseits innerhalb von 24 Stunden nach der Infusion ebenso gut aus dem Kreislauf eliminiert werden können wie die zuletzt genannten, in dieser Hinsicht vorteilhaften niedermolekularen Vergleichspräparate.

Auch die vorgenommenen Gerinnungsanalysen (plasmatische Gerinnungstests, Thrombelastographie, Bestimmung der vWF-Konzentrationen) lieferten unerwartete, weil völlig andere Ergebnisse als sie mit hochmolekularen HES-Präparaten bisher erzielt werden konnten. Während bereits mittel- und in noch viel ausgeprägtem Maß hochmolekulare Hydroxyethylstärken bisher regelmäßig eine weitaus stärkere Beeinträchtigung der Blutgerinnung im Sinne einer Hypokoagulabilität erkennen ließen als niedermolekulare HES-Lösungen (J. Treib et al., Intensive Care Med. (1999), S. 258 bis 268; R.G. Strauss et al., Transfusion (1988), S. 257 - 260), zeigten sich zwischen den erfindungsgemäßen hochmolekularen HES-Präparaten und der bekannten niedermolekularen Vergleichslösung keine signifikanten Unterschiede (vgl. Tabelle 5).

**Tabelle 5**

| **Parameter** | **Lösung** | **vor Infusion** | **nach Infusion 5 min** | **nach Infusion 1 h** | **nach Infusion 2 h** | **nach Infusion 4 h** | **nach Infusion 24h** | **p-Wert** |
|---|---|---|---|---|---|---|---|---|
| **PT** (sec) | HES 130/0,42 | 8,57 ± 0,73 | 9,16 ± 0,66 | 9,00 ± 0,70 | 9,06 ± 0,67 | 8,77 ± 0,42 | 9,30 ± 0,64 | - |
| | HES 500/0,42 | 9,32 ± 1,90 | 9,08 ± 0,49 | 8,97 ± 0,56 | 8,80 ± 0,33 | 8,57 ± 0,37 | 9,38 ± 0,58 | 0,533 |
| | HES 900/0,42 | 8,59 ± 1,06 | 8,66 ± 0,51 | 8,56 ± 0,56 | 8,40 ± 0,27 | 8,21 ± 0,27 | 9,42 ± 0,51 | 0,068 |
| **aPTT (sec)** | HES 130/0,42 | 12,19 ± 1,83 | 14,19 ± 4,21 | 13,68 ± 3,57 | 13,59 ± 1,57 | 13,11 ± 1,10 | 14,58 ± 3,01 | - |
| | HES 500/0,42 | 11,97 ± 1.00 | 13,37 ± 1,12 | 12,65 ± 0,73 | 13,04 ± 1,31 | 13,17 ± 1,31 | 13,72 ± 1,40 | 0,893 |
| | HES 900/0,42 | 11,94 ± 1,31 | 12,59 ± 1,16 | 13,10 ± 1,62 | 12,87 ± 1,06 | 12,78 ± 2,02 | 13,37 ± 1,50 | 0,706 |
| **Cl** | HES 130/0,42 | 4,53 ± 1,33 | 3,30 ± 0,91 | 4,18 ± 0,91 | 4,81 ± 0,63 | 4,48 ± 0,65 | 5,22 ± 0,35 | - |
| | HES 500/0,42 | 5,04 ± 0,81 | 2,88 ± 1,28 | 4,47 ± 0,77 | 4,71 ± 0,65 | 4,18 ± 0,98 | 5,14 ± 0,79 | 0.303 |
| | HES 900/0,42 | 5,35 ± 0,79 | 3,28 ± 1,18 | 4,47 ± 0,93 | 4,93 ± 0,82 | 4,68 ± 1,03 | 5,53 ± 0,55 | 0,468 |
| **vWF funkt..** (%) | HES 130/0,42 | 33,41 ± 10,18 | 30,09 ± 7,68 | 32,20 ± 8,63 | 33,59 ± 10,94 | 40,91 ± 10,48 | 28,80 ± 6,84 | - |
| | HES 500/0,42 | 32,11 ± 4,85 | 25,38 ± 3,84 | 27,65 ± 4,32 | 30,80 ± 4,67 | 37,94 ± 4,65 | 23,04 ± 4,19 | 0,853 |
| | HES 900/0,42 | 30,48 ± 3,95 | 24,78 ± 3,35 | 27,14 ± 4,14 | 31,41 ± 4,83 | 37,87 ± 8,06 | 24,84 ± 6,74 | 0,768 |
| **vWF antig**. (%) | HES 130/0,42 | 46,10 ± 8,45 | 35,80 ± 4,83 | 39,40 ± 8,42 | 40,10 ± 9,21 | 44,30 ± 6,80 | 38,22 ± 5,54 | - |
| | HES 500/0,42 | 42,80 ± 4,13 | 37,90 ± 7,37 | 35,50 ± 4,22 | 37,60 ± 7,41 | 39,80 ± 10,65 | 38,00 ± 8,15 | 0,499 |
| | HES 900/0,42 | 48,10 ± 19,56 | 33,30 ± 5,48 | 35,70 ± 5,89 | 37,80 ± 4,26 | 42,00 ± 4,85 | 41,67 ± 6,08 | 0,473 |

Tabelle 5 zeigt den Zeitverlauf der plasmatischen Gerinnungsparameter Prothrombinzeit (PT), aktivierte partielle Thromboplastinzeit (aPTT), funktionelle Aktivität des von Willebrand-Faktors (vWF funktionell) und antigenische Konzentration des von Willebrand-Faktors (vWF antigenisch) und Zeitverlauf des Koagulationsindex (Cl) der Thrombelastographie nach Infusion von jeweils 20 ml/kg 6 % HES 130/0,42, 6 % HES 500/0,42 bzw. 6 % HES 900/0,42 beim Schwein. Die statistische Signifikanzprüfung (Interaktion von Lösungs- und Zeiteffekt) erfolgte zwischen HES 500/0,42 und HES 900/0,42 jeweils im Vergleich zu HES 130/0,42 mittels zweiseitiger ANOVA. Trotz höherer Molekulargewichte, höherer Konzentrationen sowie längerer Verweildauer im Plasma (vgl. Figur 1 und Tabelle 4) beeinflussten die erfindungsgemäßen hochmolekularen HES-Spezies (HES 500/0,42 und HES 900/0,42) die Blutgerinnung nicht mehr als niedermolekulare HES (HES 130/0,42).

Mit anderen Worten zeigten die erfindungsgemäßen Hydroxyethylstärken trotz der durch Molekulargewichtserhöhung erreichten längeren Plasmaverweildauer nicht die Nachteile bekannter hochmolekularer HES-Lösungen, wie z.B. deren Beeinträchtigung der Blutgerinnung.

Überraschend zeigte sich zudem bei den tierexperimentellen Untersuchungen, dass die erfindungsgemäßen hochmolekularen Hydroxyethylstärken - anders als bekannte hochmolekulare Hydroxyethylstärken - die Blut- und Plasmaviskosität im Vergleich mit niedermolekularen HES nicht erhöhten. Bei niedrigen Scherkräften wurde unter den erfindungsgemäßen Hydroxyethylstärken sogar eine niedrigere Viskosität als unter der niedermolekularen HES gefunden (vgl. Tabelle 6: Plasmaviskosität).

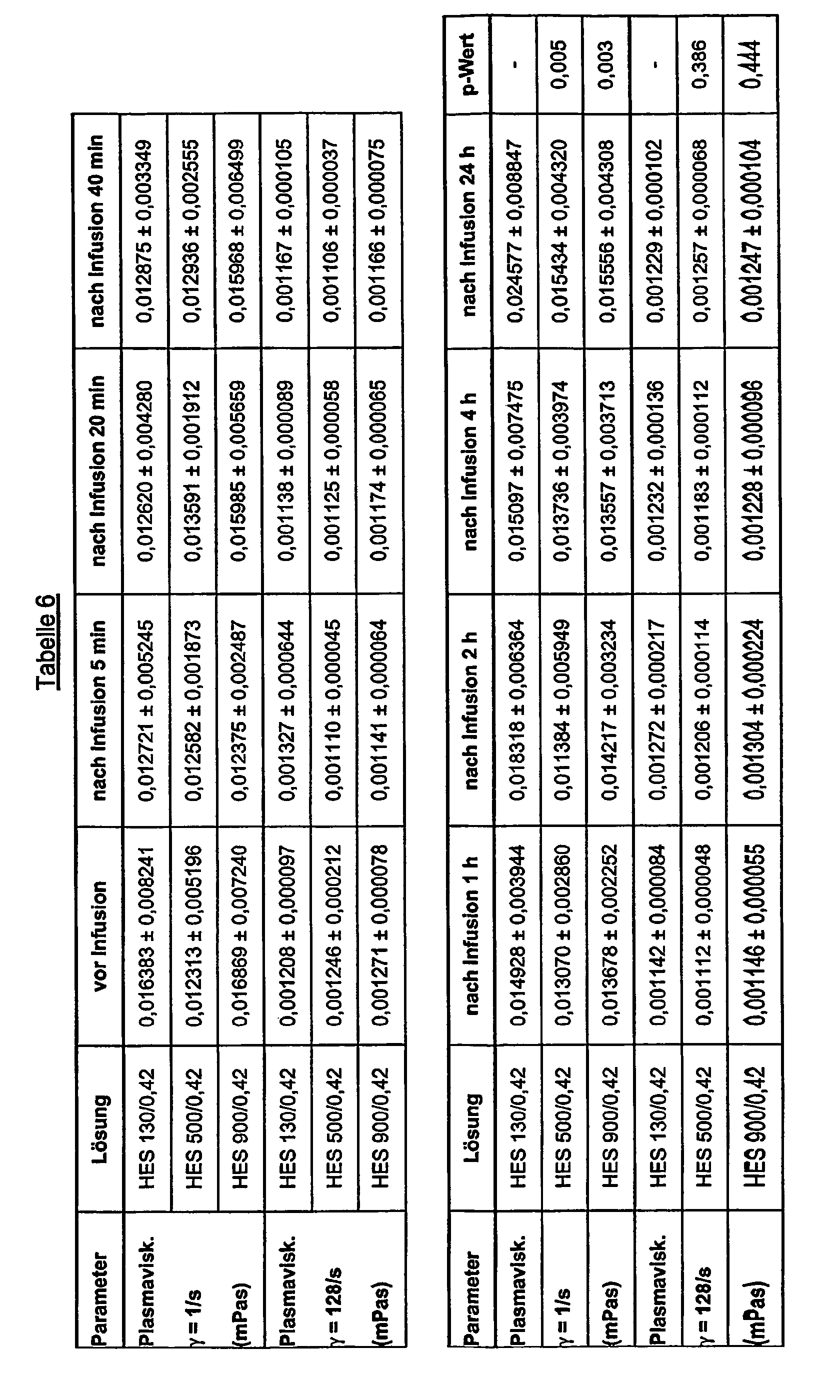

Tabelle 6 zeigt den Zeitverlauf der Plasmaviskosität bei niedrigen und hohen Scherkräften (γ = 1/s und y = 128/s nach Infusion von jeweils 20 ml/kg HES 130/0,42, 6 % HES 500/0,42 bzw. 6 % HES 900/0,42 beim Schwein. Die statistische Signifikanzprüfung (Interaktion von Lösungs- und Zeiteffekt) erfolgte zwischen HES 500/0,42 und HES 900/0,42 jeweils im Vergleich zu HES 130/0,42 mittels zweiseitiger ANOVA und ließ keine Unterschiede zwischen den hochmolekularen HES-Spezies (HES 900 und HES 500) und der niedermolekularen HES (HES 130) erkennen. Bei niedrigen Scherkräften erwies sich die Wechselwirkung von Lösungs- und Zeiteffekt bei der Plasmaviskosität unter den hochmolekularen HES-Spezies (HES 500/0,42 und HES 900/0,42) als niedriger im Vergleich zur niedermolekularen HES (HES 130/0,42). Dies war jedoch auf den Zeit- und nicht auf den Lösungseffekt zurückzuführen. Bei hohen Scherkräften bestand kein Unterschied: insbesondere war die Plasmaviskosität unter den hochmolekularen HES-Spezies (HES 900/0,42 und HES 500/0,42) nicht höher als unter niedermolekularer HES (HES 13010,42).

Die Plasmaviskosität steigt unter den erfindungsgemäßen HES-Lösungen demnach nicht an. Dass kein Anstieg der Plasmaviskosität beobachtet wurde, ist insofern überraschend, da Hydroxyethylstärke mit einem vergleichbaren, Molekulargewicht von 500000, wie in der US-A-5,502,043 offenbart (Vergleichsbeispiel 3) einen Anstieg der Plasmaviskosität aufgezeigt hat. Wenn die Viskosität nicht ansteigt, so führt dies zu einer ungestörten Kapillarperfusion (Mikrozirkulation) und einer verbesserten nutritiven Sauerstoffversorgung der Gewebe.

Neben den vorstehend beschriebenen in vivo-Untersuchungen wurden auch in vitro-Untersuchungen durchgeführt, bei denen speziell der Einfluss des C₂/C₆-Verhältnisses auf die Blutgerinnung geprüft wurde und zwar ebenfalls mit Hilfe der Thrombelastographie. Zu diesem Zweck wurden drei hochmolekulare HES-Lösungen (Mw: 800 kD) mit niedrigem MS (0.4) und niedrigem (3:1), mittlerern (7:1) bzw. hohem (12:1) C₂/C₆-Verhältnis hergestellt und folgendermaßen untersucht. Von 30 männlichen und weiblichen chirurgischen Patienten (Ausschlußkriterien: bekannte Gerinnungsstörungen, Behandlung mit Hemmstoffen der Blutgerinnung, Einnahme von Acetylsalicylsäure oder anderen nichtsteroidalen antiinflammatorischen Medikamenten innerhalb von 5 Tagen vor der Operation) wurde während der Anästhesieeinleitung eine Blutprobe entnommen. In jeder Blutprobe wurde die Gerinnung mittels Thrombelastographie gemessen und zwar im unverdünnten Blut und nach in vitro-Hämodilution (20 %, 40 % und 60 %) mit jeder der 3 HES-Lösungen (HES 800/0,4/3:1; HES 800/0,4/7:1 bzw. HES 800/0,4/12:1). Bestimmt wurde - wie schon bei den in vivo-Untersuchungen - der Coagulation Index (Cl), der die einzelnen Partialfunktionen der Thrombelastographie zusammenfasst. Die Mittelwerte (± SD) der gefundenen Cl-Werte sind in der nachfolgenden Tabelle 7 dargestellt und zwar als Abweichung vom Cl in der jeweiligen unverdünnten Blutprobe.

**Tabelle 7**

| | HES | HES | HES |
|---|---|---|---|
| | 800/0,4/3:1 | 800/0,4/7:1 | 800/0,4/12:1 |
| 20 % | - 2,32 | - 2,86 | - 3,35 |
| Hämodilution | (1,20) | (1,22) | (1,07) |
| 40 % | - 5,91 | - 6,17 | - 6,50 |
| Hämodilution | (1,68) | (2,03) | (1,69) |
| 60 % | - 9,71 | -10,37 | -10,55 |
| Hämodilution | (2,70) | (3,05) | (2,60) |

Bei allen Hämodilutionsstufen wurde der Cl in bezug auf den Cl des Nativbluts umso weniger gesenkt, die Blutgerinnung also umso weniger beeinflußt, je niedriger das C₂/C₆-Verhältnis der zur Hämodilution verwendeten HES lag. Der Lösungseffekt zwischen den Hämodilutionsserien war signifikant unterschiedlich (p < 0,05; ANOVA). Die Resultate zeigen, dass eine Erniedrigung des C₂/C₆-Verhältnisses von Hydroxyethylstärken günstig für deren Einfluss auf die Blutgerinnung ist und zwar in dem Sinn, dass die Blutgerinnung bei niedrigem C₂/C₆-Verhältnis weniger gehemmt wird als bei hohem. Dies ist insofern von Bedeutung, als HES-Lösungen unter anderem als Plasmaersatzmittel nach traumatisch oder operativ bedingten Blutungen eingesetzt werden und in dieser Situation den Blutverlust nicht durch eine Hemmung der Blutgerinnung noch zusätzlich vergrößern dürfen. Die Ergebnisse der vorstehend beschriebenen Untersuchung zeigen weiterhin, dass von dem C₂/C₆-Verhältnis von HES-Lösungen eine Eigenwirkung auf die Blutgerinnung ausgeht, die von anderen molekularen Parametern der HES und ihrem Verhalten im Kreislauf unabhängig ist. Dies war bisher nicht bekannt.

## Patentansprüche

1. Hydroxyethylstärke mit einem mittleren Molekulargewicht Mw größer oder gleich 500000, **dadurch gekennzeichnet, dass** der molare Substitutionsgrad MS zwischen 0,25 und 0,5 ist und das C₂/C₆-Verhältnis 2 bis unterhalb von 8 beträgt.

2. Hydroxyethylstärke nach Anspruch 1, **dadurch gekennzeichnet, dass** der molare Substitutionsgrad MS 0,35 bis 0,5, vorzugsweise 0,39 bis kleiner oder gleich 0,45, insbesondere größer 0,4 bis 0,44 ist.

3. Hydroxyethylstärke nach einem der Ansprüche 1 und 2, **dadurch gekennzeichnet, dass** das mittlere Molekulargewicht oberhalb 600000 bis 1500000, vorzugsweise 620000 bis 1200000, besonders bevorzugt von 700000 bis 1000000 beträgt.

4. Hydroxyethylstärke nach einem der Ansprüche 1 bis 3, **dadurch gekennzeichnet, dass** das C₂/C₆-Verhältnis 2 bis 7, vorzugsweise 2,5 bis kleiner oder gleich 7, besonders bevorzugt 2,5 bis 6 und insbesondere bevorzugt 4 bis 6 ist.

5. Hydroxyethylstärke nach einem der Ansprüche 1 bis 4, **dadurch gekennzeichnet, dass** sie aus einer Wachsmaisstärke erhältlich ist.

6. Pharmazeutische Zubereitung umfassend eine Hydroxyethylstärke nach einem der Ansprüche 1 bis 5.

7. Pharmazeutische Zubereitung nach Anspruch 6, **dadurch gekennzeichnet, dass** sie als wässrige Lösung oder als kolloidale wässrige Lösung vorliegt.

8. Pharmazeutische Zubereitung nach einem der Ansprüche 6 oder 7, **dadurch gekennzeichnet, dass** die Hydroxyethylstärke in einer Konzentration von bis zu 20 %, vorzugsweise 0,5 bis 15 %, besonders bevorzugt 2 bis 12 %, insbesondere 4 bis 10 %, beispielsweise 6 % vorliegt.

9. Pharmazeutische Zubereitung nach einem der Ansprüche 6 bis 8, **dadurch gekennzeichnet, dass** sie zusätzlich Natriumchlorid, vorzugsweise in 0,9 prozentiger Konzentration enthält.

10. Pharmazeutische Zubereitung nach einem der Ansprüche 6 bis 9, **dadurch gekennzeichnet, dass** sie zusätzlich plasmaadaptierte Elektrolyte aufweist.

11. Pharmazeutische Zubereitung nach einem der Ansprüche 6 bis 10, **dadurch gekennzeichnet, dass** sie als gepufferte Lösung und/oder als Lösung mit metabolisierbaren Anionen vorliegt.

12. Pharmazeutische Zubereitung nach einem der Ansprüche 6 bis 11, **dadurch gekennzeichnet, dass** sie als hypertone Lösung vorliegt.

13. Pharmazeutische Zubereitung nach einem der Ansprüche 6 bis 12, **dadurch gekennzeichnet, dass** sie sterilfiltriert oder hitzesterilisiert ist.

14. Pharmazeutische Zubereitung nach einem der Ansprüche 6 bis 13, **dadurch gekennzeichnet, dass** sie ein Volumenersatzmittel ist.

15. Pharmazeutische Zubereitung nach einem der Ansprüche 6 bis 14, **dadurch gekennzeichnet, dass** sie einen pharmazeutischen Wirkstoff oder eine Wirkstoffkombination enthält.

16. Verwendung einer pharmazeutischen Zubereitung nach einem der Ansprüche 6 bis 15 zur Herstellung eines Plasmaersatzmittels oder eines Plasmaexpanders.

17. Verfahren zur Herstellung einer Hydroxyethylstärke, vorzugsweise wie in einem der Ansprüche 1 bis 5 definiert durch
(i) Umsetzung von in Wasser suspendierter Stärke, vorzugsweise Maisstärke, mit Ethylenoxid und
(ii) anschließender Teilhydrolysierung des erhaltenen Stärkederivats mit Säure, vorzugsweise Salzsäure bis zum gewünschten mittleren Molekulargewichtsbereich der Hydroxyethylstärke.

18. Verfahren nach Anspruch 17, **dadurch gekennzeichnet, dass** der in Wasser suspendierten Stärke ein Alkalisierungsmittel, vorzugsweise NaOH, zugesetzt wird.

19. Verfahren nach einem der Ansprüche 17 oder 18, **dadurch gekennzeichnet, dass** der suspendierten Stärke Alkalisierungsmittel in einer Menge zugesetzt wird, so dass das molare Verhältnis von Alkalisierungsmittel zu Stärke größer 0,2, vorzugsweise 0,25 bis 1, insbesondere 0,3 bis 0,8 ist.

20. Verfahren nach einem der Ansprüche 17 bis 19, **dadurch gekennzeichnet, dass** das Verfahren zusätzlich die Schritte (iii) der Sterilisation und gegebenenfalls (iv) der Ultrafiltration umfasst.

21. Verwendung der pharmazeutischen Zubereitung nach einem der Ansprüche 6 bis 15 zur Herstellung eines Medikaments zur Aufrechterhaltung der Normovolämie und/oder zur Verbesserung der Makro- und Mikrozirkulation und/oder zur Verbesserung der nutritiven Sauerstoffversorgung und/oder zur Stabilisierung der Hämodynamik und/oder zur Verbesserung der Volumenwirksamkeit und/oder zur Verringerung der Plasmaviskosität und/oder zur Erhöhung der Anämietoleranz und/oder zur Hämodilution, insbesondere zur therapeutischen Hämodilution bei Durchblutungsstörungen und arteriellen, speziell peripheren arteriellen, Verschlusskrankheiten.

22. Kit umfassend in getrennter Form
(i) die Hydroxyethylstärke, wie in den Ansprüchen 1 bis 5 definiert,
(ii) sterile Salzlösung, vorzugsweise sterile Natriumchlorid-Lösung und gegebenenfalls
(iii) einen pharmazeutischen Wirkstoff oder eine Wirkstoffkombination.

23. Kit gemäß Anspruch 22, **dadurch gekennzeichnet, dass** die Einzelkomponenten (i), (ii) und gegebenenfalls (iii) in getrennten Kammern in einem Mehrkammerbeutel vorliegen.

## Claims

1. A hydroxyethylstarch having an average molecular weight, Mw, of greater than or equal to 500,000, **characterized by** having a molar substitution MS of from 0.25 to 0.5 and a C₂/C₆ ratio of from 2 to less than 8.

2. The hydroxyethylstarch according to claim 1, **characterized in that** said molar substitution MS is from 0.35 to 0.5, preferably from 0.39 to less than or equal to 0.45, especially from greater than 0.4 to 0.44.

3. The hydroxyethylstarch according to either of claims 1 and 2, **characterized in that** said average molecular weight is from above 600,000 to 1,500,000, preferably from 620,000 to 1,200,000, more preferably from 700,000 to 1,000,000.

4. The hydroxyethylstarch according to any of claims 1 to 3, **characterized in that** said C₂/C₆ ratio is from 2 to 7, preferably from 2.5 to less than or equal to 7, more preferably from 2.5 to 6, even more preferably from 4 to 6.

5. The hydroxyethylstarch according to any of claims 1 to 4, **characterized by** being obtainable from a waxy maize starch.

6. A pharmaceutical formulation containing a hydroxyethylstarch according to any of claims 1 to 5.

7. The pharmaceutical formulation according to claim 6, **characterized by** being in the form of an aqueous solution or of a colloidal aqueous solution.

8. The pharmaceutical formulation according to either of claims 6 or 7, **characterized in that** said hydroxyethylstarch is present in a concentration of up to 20%, preferably from 0.5 to 15%, more preferably from 2 to 12%, especially from 4 to 10%, for example, 6%.

9. The pharmaceutical formulation according to any of claims 6 to 8, **characterized by** additionally containing sodium chloride, preferably in a concentration of 0.9%.

10. The pharmaceutical formulation according to any of claims 6 to 9, **characterized by** additionally including plasma-adapted electrolytes.

11. The pharmaceutical formulation according to any of claims 6 to 10, **characterized by** being in the form of a buffered solution and/or of a solution with metabolizable anions.

12. The pharmaceutical formulation according to any of claims 6 to 11, **characterized by** being in the form of a hypertonic solution.

13. The pharmaceutical formulation according to any of claims 6 to 12, **characterized by** being sterile filtered or heat sterilized.

14. The pharmaceutical formulation according to any of claims 6 to 13, **characterized by** being a volume replacement.

15. The pharmaceutical formulation according to any of claims 6 to 14, **characterized by** containing a pharmaceutically active ingredient or a combination of active ingredients.

16. Use of a pharmaceutical formulation according to any of claims 6 to 15 for the preparation of a plasma replacement or plasma expander.

17. A process for the preparation of a hydroxyethylstarch, preferably one as defined in any of claims 1 to 5, by:
(i) reacting water-suspended starch, preferably corn starch, with ethylene oxide; and
(ii) then partially hydrolyzing the starch derivative obtained with acid, preferably hydrochloric acid, until the desired range of average molecular weight of the hydroxyethylstarch has been reached.

18. The process according to claim 17, **characterized in that** an alkalizing agent, preferably NaOH, is added to said water-suspended starch.

19. The process according to either of claims 17 or 18, **characterized in that** an alkalizing agent is added to said suspended starch in such an amount that the molar ratio of alkalizing agent to starch is above 0.2, preferably from 0.25 to 1, especially from 0.3 to 0.8.

20. The process according to any of claims 17 to 19, **characterized by** additionally comprising the steps of (iii) sterilization and, optionally, (iv) ultrafiltration.

21. Use of the pharmaceutical formulation according to any of claims 6 to 15 the manufacture of a medicament for maintaining normovolemia and/or for improving the macro- and microcirculation and/or for improving the nutritive oxygen supply and/or for stabilizing hemodynamics and/or for improving the volume efficiency and/or for reducing the plasma viscosity and/or for increasing anemia tolerance and/or for hemodilution, especially for therapeutic hemodilution in disturbed blood supply and arterial, especially peripheral arterial, occlusive diseases.

22. A kit comprising separately:
(i) the hydroxyethylstarch as defined in claims 1 to 5;
(ii) a sterile salt solution, preferably sodium chloride solution; and optionally
(iii) a pharmaceutically active ingredient or a combination of active ingredients.

23. The kit according to claim 22, **characterized in that** the individual components (i), (ii) and optionally (iii) are present in separate compartments in a multicompartment bag.

## Revendications

1. Hydroxyéthylamidon ayant une masse moléculaire moyenne Mw supérieure ou égale à 500000, **caractérisé en ce que** le degré de substitution molaire MS est entre 0,25 et 0,5 et le rapport C₂/C₆ est 2 à inférieur à 8.

2. Hydroxyéthylamidon selon la revendication 1 **caractérisé en ce que** le degré de substitution molaire MS est 0,35 à 0,5, de préférence 0,39 à inférieur ou égal à 0,45, en particulier supérieur à 0,4 à 0,44.

3. Hydroxyéthylamidon selon l'une des revendications 1 et 2 **caractérisé en ce que** la masse moléculaire moyenne est supérieure à 600000 à 1500000, de préférence 620000 à 1200000, de manière particulièrement préférée de 700000 à 1000000.

4. Hydroxyéthylamidon selon l'une des revendications 1 à 3 **caractérisé en ce que** le rapport C₂/C₆ est 2 à 7, de préférence 2,5 à inférieur ou égal à 7, de manière particulièrement préférée 2,5 à 6 et de manière spécialement préférée 4 à 6.

5. Hydroxyéthylamidon selon l'une des revendications 1 à 4 **caractérisé en ce qu'**il peut être obtenu à partir d'un amidon de maïs cireux.

6. Préparation pharmaceutique comprenant un hydroxyéthylamidon selon l'une des revendications 1 à 5.

7. Préparation pharmaceutique selon la revendication 6 **caractérisée en ce qu'**elle est sous forme de solution aqueuse ou de solution aqueuse colloïdale.

8. Préparation pharmaceutique selon l'une des revendications 6 ou 7 **caractérisée en ce que** l'hydroxyéthylamidon est présent en une concentration de jusqu'à 20 %, de préférence de 0,5 à 15 %, de manière particulièrement préférée de 2 à 12 %, en particulier de 4 à 10 %, par exemple de 6 %.

9. Préparation pharmaceutique selon l'une des revendications 6 à 8 **caractérisée en ce qu'**elle contient en outre du chlorure de sodium, de préférence en une concentration de 0,9 pourcent.

10. Préparation pharmaceutique selon l'une des revendications 6 à 9 **caractérisée en ce qu'**elle comporte en outre des électrolytes adaptés au plasma.

11. Préparation pharmaceutique selon l'une des revendications 6 à 10 **caractérisée en ce qu'**elle est sous forme de solution tamponnée et/ou sous forme de solution avec des anions métabolisables.

12. Préparation pharmaceutique selon l'une des revendications 6 à 11 **caractérisée en ce qu'**elle est sous forme de solution hypertonique.

13. Préparation pharmaceutique selon l'une des revendications 6 à 12 **caractérisée en ce qu'**elle est filtrée par filtration stérile ou stérilisée à la chaleur.

14. Préparation pharmaceutique selon l'une des revendications 6 à 13 **caractérisée en ce que** c'est un agent de remplacement volumique.

15. Préparation pharmaceutique selon l'une des revendications 6 à 14 **caractérisée en ce qu'**elle contient un principe actif pharmaceutique ou une combinaison de principes actifs.

16. Utilisation d'une préparation pharmaceutique selon l'une des revendications 6 à 15 pour la production d'un agent de remplacement du plasma ou d'un extendeur du plasma.

17. Procédé de production d'un hydroxyéthylamidon, de préférence défini comme dans l'une des revendications 1 à 5 par
(i) réaction d'amidon en suspension dans l'eau, de préférence d'amidon de maïs, avec de l'oxyde d'éthylène et
(ii) hydrolyse partielle subséquente du dérivé d'amidon obtenu avec un acide, de préférence l'acide chlorhydrique jusqu'au domaine souhaité de masse moléculaire moyenne de l'hydroxyéthylamidon.

18. Procédé selon la revendication 17 **caractérisé en ce qu'**un agent d'alcalinisation, de préférence NaOH, est ajouté à l'amidon en suspension dans l'eau.

19. Procédé selon l'une des revendications 17 ou 18 **caractérisé en ce qu'**un agent d'alcalinisation est ajouté à l'amidon en suspension en une quantité telle que le rapport molaire de l'agent d'alcalinisation à l'amidon est supérieur à 0,2, de préférence 0,25 à 1, en particulier 0,3 à 0,8.

20. Procédé selon l'une des revendications 17 à 19 **caractérisé en ce que** le procédé comprend en outre les étapes (iii) de stérilisation et éventuellement (iv) d'ultrafiltration.

21. Utilisation de la préparation pharmaceutique selon l'une des revendications 6 à 15 pour la production d'un médicament pour maintenir la normovolémie et/ou pour améliorer la macro- et microcirculation et/ou pour améliorer l'apport d'oxygène nutritif et/ou pour stabiliser l'hémodynamique et/ou pour améliorer l'efficacité volumique et/ou pour réduire la viscosité du plasma et/ou pour augmenter la tolérance à l'anémie et/ou pour l'hémodilution, en particulier pour l'hémodilution thérapeutique dans le cas de troubles de l'irrigation sanguine et de maladies d'obturation artérielles, spécialement artérielles périphériques.

22. Kit comprenant sous forme séparée
(i) l'hydroxyéthylamidon, tel que défini dans les revendications 1 à 5,
(ii) une solution salée stérile, de préférence une solution de chlorure de sodium stérile et éventuellement
(iii) un principe actif pharmaceutique ou une combinaison de principes actifs.

23. Kit selon la revendication 22 **caractérisé en ce que** les différents composants (i), (ii) et éventuellement (iii) sont présents dans des chambres séparées dans un sachet à plusieurs chambres.
